# EUROPEAN PATENT SPECIFICATION

(11) **EP 0 515 660 B1**
(45) Date of publication and mention of the grant of the patent: **26.03.1997**
(21) Application number: 92902628.4
(22) Date of filing: 20.12.1991
(51) Int. Cl.: C12Q 1/68

(54) **HLA DQBETA DNA TYPING**
KARAKTERISIERUNG VON HLA-DQBETA-DNS
TYPAGE D'ADN HLA DQBETA

(30) Priority: 21.12.1990 US 632180
(43) Date of publication of application: 02.12.1992
(73) Proprietor: F. HOFFMANN-LA ROCHE AG, 4002 Basel (CH)
(72) Inventor: ERLICH, Henry, A., Oakland, CA 94602 (US); BUGAWAN, Teodorica, San Leandro, CA 94579 (US)
(74) Representative: Wächter, Dieter Ernst, Dr.
(86) International application number: US9109796
(87) International publication number: WO9211389

(56) References cited:
- EP-A- 0 237 362
- EP-A- 0 314 500
- WO-A-89/04875
- WO-A-89/11547
- US-A- 4 965 189
- PROCEEDINGS OF THE NATL. ACADEMY OF SCIENCES USA, vol. 85, May 1988, Washington, DC (US); S.J. SCHARF et al., pp. 6504-6508/

## Description

### Background of the Invention

### Field of the Invention

This invention relates to methods and compositions for determining the HLA DQB (DQbeta or DQβ) genotype of an individual. In a preferred embodiment, the invention relates to gene amplification methods disclosed and claimed in U.S. Patent Nos. 4,683,202;4,683,195; and 4,965,188. The methods and probes of the invention specifically relate to the detection of the polymorphic alleles of the Class II HLA DQB1 gene. Particularly, the presence or absence of particular HLA DQB1 alleles serve as an indicator of susceptibility to insulin-dependent diabetes mellitus (IDDM). Thus, the invention relates to the fields of molecular biology, diagnostic medicine, and forensics.

### Description of the Related Art

The Class II loci of the human major histocompatibility complex encode the HLA D cell surface glycoproteins which are expressed on B lymphocytes, activated T lymphocytes, macrophages, and dendritic cells. These proteins, which are individually designated DR, DQ, and DP, are heterodimers composed of an alpha and a highly polymorphic beta subunit and are responsible for the presentation of antigens to T cells. The variability in the highly polymorphic beta subunit is localized to the amino-terminal extracellular domain, which is thought to interact with the T cell receptor and antigen peptide fragments. Recognition of the HLA glycoprotein/peptide fragment complex by the T cell receptor of CD4 lymphocytes leads to T cell activation (Babbit et al., 1985, Nature 317:359-361; Buus et al., 1987, Science 235:1353-1358; Guillet et al., 1987, Science 235:865-870; and Sette et al., 1987, Nature 328:395-399). The genes encoding the Class II HLA proteins are located on the short arm of chromosome six in humans. These genes are highly polymorphic with most of the sequence defined polymorphism being localized to the second exon. The structures, sequences, and polymorphisms in the HLA D region have been reviewed in Trowsdale et al., 1985, Immunol. Rev. 85:5-43.

The polymorphism of the HLA D region gene product has, in general, been defined by serologic typing reagents and by the mixed lymphocyte culture (MLC) reaction in which T cell proliferation in response to homozygous typing cells (HTC) is measured in culture. Polymorphism at the HLA DQB1 locus has been detected using serologic typing reagents that define the specificities DQw1, 2, 3, and 4 (WHO Nomenclature Committee, 1990). The specificity DQw1 has been subdivided recently into the serologic subtypes DQw5 and 6, and DQw3 has been subdivided into DQw7, 8, and 9. In routine typing, however, only the DQw1, DQw2, and DQw3 specificities are distinguished.

Cellular (see Odum et al., 1987, Tissue Antigens 29:101-109), biochemical (see Lotteau et al., 1987, Immunogenetics 25:403-407), and restriction fragment length polymorphism (RFLP, see Hyldig-Nielsen et al., 1987, Proc. Natl. Acad. Sci. USA 84:1644-1648 and U.S. Patent No. 4,582,788) analyses have indicated that the degree of polymorphism in the HLA region may be more extensive than serological and immunological methods indicated. The RFLP approach is useful for identification of seven DQ specificities.

However, the RFLP technique has certain limitations. An allele carrying a variant sequence is identifiable only if the variant nucleotide is within the recognition site of a restriction enzyme used in the analysis or if a polymorphic restriction site is in linkage disequilibrium with a specific coding sequence variation. In addition, RFLP analysis simply provides evidence that a coding sequence variation exists but does not provide information on the exact nature of the variation. Moreover, the method is labor intensive and requires large amounts of high molecular weight DNA. This latter requirement often rules out the use of samples which have been kept under conditions that cause the degradation of genomic DNA.

Accurate DQ typing will be important in several medical applications, particularly in the field of organ transplantation. Typing is also useful in the study of the molecular basis of disease susceptibility. In particular, specific alleles of DQB1 and DQA1 serve as indicators for susceptibility to insulin-dependent diabetes mellitus (IDDM, Todd et al., 1987, Nature 329:559-604; Horn et al., 1988, Proc. Natl. Acad. Sci. USA 85:6012-6016; and Todd et al., 1989, Nature 338:587.)

The advent of the polymerase chain reaction (PCR) has facilitated the analysis and manipulation of complex genomic DNA. The PCR process enables one to amplify a specific sequence of nucleic acid starting from a very complex mixture of nucleic acids. This process is more fully described in U.S. Patent Nos. 4,683,195; 4,683,202; 4,889,818; and 4,965,188, and European Patent Publication Nos. 237,362 and 258,017.

The PCR process has also facilitated typing the Class II HLA DNA of an individual. Scientists have studied the polymorphic second exon of DRB loci in genomic DNA by designing oligonucleotide primers and using those primers to amplify the sequences of interest. See the article entitled "Sequence analysis of the HLA DRB and HLA DQB loci from three Pemphigus vulgaris patients" by Scharf et al., 1988, Hum. Immunol. 22:61-69. PCR based methods for HLA DP typing are described in copending Serial No. 347,506, filed May 4, 1989, and for HLA DR typing in Serial No. 623,098, filed December 6, 1990.

When the primers contain restriction enzyme recognition sequences, the amplified DNA can be cloned directly into sequencing vectors, and the nucleotide sequence of the amplification product can be readily determined. See the article entitled "Direct cloning and sequence analysis of enzymatically amplified genomic sequences" by Scharf et al., 1986, Science 233: 1076-1078.

The amplified DNA can also be studied by detection methods that employ sequence-specific oligonucleotide (SSO) probes. See the article entitled "Analysis of enzymatically amplified beta-globin and HLA DQα DNA with allele-specific oligonucleotide probes" by Saiki et al., 1986, Nature 324:163-166. The study also describes a dot blot technique to detect probe hybridization to the sample nucleic acid.

The present invention meets the need for an efficient, informative DQB1 DNA typing method by providing novel processes and reagents. These processes and reagents have led to the discovery of previously unknown DQB1 alleles that can also be typed and identified by the present method.

The present typing system can be used to type cDNA synthesized from mRNA and to type and study the expression of DQB1 alleles in tissues, transgenic systems, disease states, and cells lines. Cells that do not express the DQ antigens or show unusual seroreactivity, such as tumor cells, can be readily typed. Moreover, samples from unusual sources, e.g., ancient DNAs or forensic samples, can be typed, even when the DNA sample is degraded or when only very small quantities are available for analysis.

Because PCR can amplify a fragment of target DNA over a million-fold, and because the present system can employ PCR-generated nucleic acid, radioactively labeled probes are not necessary, and nonisotopic SSOs covalently coupled to non-radioactive labels such as horseradish peroxidase (HRP) provide sufficient sensitivity for detection. The presence of the specifically bound HRP-labeled probe can be detected in a simple dot blot format by chromogenic dye or chemiluminescent substrates in a matter of minutes.

### Summary of the Invention

The present invention provides locus-specific amplification primers and nonisotopic SSO probes that together provide a rapid, simple and precise system for typing the HLA DQB1 alleles, including those that cannot be distinguished by serological methods.

In one aspect, the present invention provides a method for determining the DQB1 type of nucleic acids in a sample, which method comprises (a) amplifying DQB1 gene second exon sequences in the presence of a primer specific for all of the alleles of the DQB1 gene and which primer does not amplify alleles of closely related genes such as DQB2 (DXB) or the DPB1 and DRB genes; (b) hybridizing any amplified nucleic acid with a set of oligonucleotide probes under conditions such that only probes hybridized to exactly complementary sequences can remain stably bound to the amplified DNA; and (c) determining whether amplification has occurred. The said probes can be labeled, with either radioactive or nonradioactive labels, or attached to a solid support, or both.

In another aspect, the present invention provides oligonucleotide primers for use in the present method. These primers are specific for all of the alleles of the DQB1 gene and do not amplify alleles of closely related genes such as DQB2 (DXB) or the DPB1 and DRB genes. The preferred primers are selected from the group consisting of DB130 (SEQ ID NO:47) and DB131 (SEQ ID NO:48). The most preferred primers are the primer pair DB130 (SEQ ID NO:47)/DB131 (SEQ ID NO:48) or the primer pair DB130 (SEQ ID NO:47) and GH29 (SEQ ID NO:70).

In a final aspect, the present invention provides kits for practising the present invention. Such a kit comprise a primer of the invention. Optionally, a kit can further comprise oligonucleotide probes for detecting the presence and determining the type of DQB1 alleles in a sample and amplification reagents. If the amplification method of choice is PCR, such reagents include a thermostable polymerase and/or deoxyribonucleoside triphosphates.

### Brief Description of the Drawings

Figure 1 shows the analysis of DQB1 and DQB2 coamplification, as described in Example 2.

Figure 2 shows the results of the non-radioactive HLA DQB1 typing system, as described in Example 4.

### Detailed Description of the Invention

The present invention provides an HLA DQB1 typing system using novel primers that facilitate accurate typing as defined in the appended set of claims. The invention can be used to type heterozygous samples from a variety of sources, including cDNA templates, and can be used to detect allelic variants not distinguishable by serological methods. This typing system preferably utilizes a dot blot format that is simple and rapid to perform, produces detectable signals in minutes, and will prove valuable for tissue typing and determining individual identity and disease susceptibility. The method is also suitable for identifying previously unreported DQB1 alleles.

In a preferred embodiment, the invention provides a method for distinguishing between any of 15 HLA DQB1 alleles present in a sample. In this method, only 16 SSOs and one primer pair are required. These compositions and methods enable resolution of 117 of the 120 possible DQB1 genotypes. Preferably, the SSOs are horseradish peroxidase conjugated-SSOs (HRP-SSOs).

The diversity of DQB loci and the large number of alleles at these loci in the population make difficult the process of identifying the particular DQB loci and alleles from which a nucleic acid in a sample originates. The present invention allows one to make this determination with great specificity and so can be used to identify the particular individual from whom a sample was taken. This discrimination power in turn leads to the applications of the invention in the field of forensic science.

Because PCR, or other amplification processes, can be used to amplify very small amounts of DNA (or degraded DNA), the present invention can be used to type HLA DQB1 from unusual sources such as a buccal swab, a single hair, and even DNA from preserved ancient specimens, so as to allow analysis of the alleles from prehistoric sources, e.g., early hominids.

Prior to the invention, sequence analysis of human DQB1 alleles had identified 15 different amino acid sequences (Erlich et al., 1991, Diabetes 40(4):478-481, Bugawan et al., 1991, Immunogenetics 33:163-170, Horn et al., 1988, Proc. Natl. Acad. Sci. USA 85:6012-6016; Scharf et al., 1989, Proc. Natl. Acad. Sci. USA 86:6215-6219; and Fronek et al., 1988, Am. J. Med. 85(Suppl 6A):42-44). PCR amplification has also made possible the phylogenetic analysis of DQβ polymorphism in primates (Gyllensten and Erlich, 1989, Proc. Natl. Acad. Sci. USA 86:9486-9990), revealing four major allelic lineages or types: DQβ1a (corresponding to DQw5), DQβ1b (corresponding to DQw6), DQβ2, DQβ3, and DQβ4. These allelic types preceded speciation and were present in the ancestral species that gave rise to the hominoid lineages. The 15 human DQB1 alleles currently known appear to have undergone more recent diversification within these ancient lineages or allelic types (Gyllensten and Erlich, 1990, Proc. Natl. Acad. Sci. USA 87:1835-1839).

The allelic sequence diversity at the DQB1 locus is considerably greater (15 alleles) than the number of currently detectable serologic specificities (n=7). In particular, there are nine different DQB1 alleles that type serologically as DQw1 and as the subtypes DQw5 or Dw6. Polymorphism not detectable by serology can have profound biological significance. For example, DQB1*0503 (Asp-57) and *0501 (Val-57) and *0502 (Ser-57) differ by only one residue and type as DQw5 but DQB1*0503 confers a high risk for Pemphigus vulgaris while the other alleles do not (Scharf et al., 1988, supra). The present simple, rapid and precise method of typing DQB1 polymorphism will be valuable in the areas of disease susceptibility, tissue transplantation, individual identification, and anthropological genetics.

The research potential of the present invention should in no way obscure the immediate clinical applications. The genes and gene products of the MHC play a central role in the immunological state of an individual, and particular MHC gene products are associated with disease resistance and susceptibility. Because the present invention allows the determination of the MHC DQB1 gene products in a sample, the invention also has applications in the field of medicine, particularly for diagnostic methods.

The present method is suitable for DNA typing samples that contain no DQB1 protein products. Because one can perform DNA typing by amplifying cDNA synthesized from mRNA, the present method facilitates the study of the expression of HLA DQB1 from various cells or tissues and can be used to determine if there is an association between HLA DQB1 expression and susceptibility to transformation, autoimmunity, or other health conditions.

In another aspect, the present invention is suitable for determining susceptibility to IDDM. IDDM is a chronic autoimmune disease in which the regulation of glucose metabolism is dysfunctional due to the immunological destruction of the pancreatic islet beta cells which produce insulin (Eisenbarth, 1986, N. Engl. J. Med. 314:1360-1368). IDDM, as well as many other autoimmune diseases, has been associated with serologically defined alleles of the HLA Class II antigens (Svejgaard et al., 1983, Immunol. Rev. 70:193-218, and Tiwari and Terasaki, HLA and Disease Associations [1985, Springer, New York, NY]; USP 4,965,189 and EP-A-314,500).

In population studies, the serological types HLA-DR3 and DR4 are positively associated and DR2 is negatively associated with IDDM. In addition, the analysis of patterns of HLA haplotype sharing among affected sibling pairs (Thompson and Bodmer, pp. 84-93, eds., HLA and Disease, Dausset and Svejgaard, Copenhagen, Munksgaard, 1977), and disease and HLA linkage in families, has implicated loci in the HLA region on chromosome 6 in IDDM susceptibility. To date, the unusual DR2 haplotypes identified in rare DR2 IDDM patients differ at both the HLA DRB1 and DQB1 loci from the common DR2 haplotypes that confer resistance to diabetes (Todd et al., 1987, Nature 329:599-604; Horn et al., 1988, Proc. Natl. Acad. Sci. USA 85:6012-6016; and Erlich et al., 1990, Diabetes 39:96-103).

The present invention provides means for discerning the role of the DQB1 locus in IDDM resistance. Example 5 describes the application of the present method for characterizing the second exon of the DQB1 genes of a particularly unusual IDDM family. The example also describes the analysis of DR1 and DR2 haplotypes (see Erlich et al., 1991, Diabetes 40(4):478-481). In this family, all three siblings who inherited the unusual maternal DR1 and unusual paternal DR2 haplotypes have IDDM, demonstrating that the combination of these two haplotypes is responsible for the high penetrance of diabetes seen in this family.

The application of the present methods and compositions provides means for advancing the understanding of disease susceptibility factors. In addition, the discriminating power of this system will be valuable in typing potential transplantation donors, where very precise HLA DQB1 matching is critical in minimizing risk of rejection or graft versus host disease. See the related article entitled "Mixed lymphocyte reactions for individuals with phenotypic identity for specific HLA B, DR determinants: The role of linkage disequilibrium and of specific DR and other Class II determinants" by Pollack et al., 1983, J. Clin. Immunol. 3:341-351. Disease susceptibility studies have shown that single nucleotide differences in the DQβ alleles can be medically significant. See the article entitled "Specific HLA DQβ and HLA DRβ1 alleles confer susceptibility to Pemphigus vulgaris" by Scharf et al., 1989, Proc. Natl. Acad. Sci. USA 86:6215-6219.

In addition to the above benefits, the present invention also provides methods for identifying previously unknown DQB1 alleles, and related primers, probes, and methods for the identification of any DQB1 allele. Unusual patterns of SSO probe hybridization using this typing system identify new alleles, as shown in Example 5.

The present invention provides a number of DQB primers for amplifying nucleic acid segments to facilitate accurate typing. These primers are shown in Table 1 below.

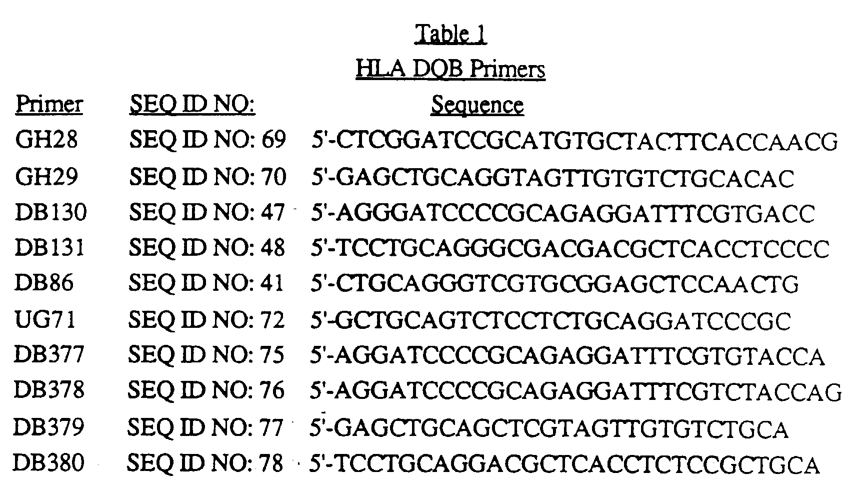

The nucleotide and amino acid sequences of 15 DQB1 alleles and the DQB2 (DXB) locus allele are provided in the Sequence Listing section. The sequence identifiers for the nucleotide and amino acid sequences of each allele are given below. Tables 2 and 4, below, provide equivalent sequence information in a format which facilitates visual comparisons.

Some of the 15 HLA DQB1 alleles, defined as amino acid sequences, have subtypes based on silent polymorphisms. For example, two different DQB1*0503 (previously DQβ1.3) nucleotide sequences (GAC vs GAT for the Asp codon at position 57; see Scharf et al., 1989, supra.) and two different DQB1*0301 (GCG vs GCA for the Ala codon at position 38) have been identified. These subtypes are detected by the probes disclosed in the present methods. In addition, two DQB1*0303 nucleotide sequences (see "Nomenclature for factors of the HLA system", 1990, Immunogenetics 33:301-309) have been reported.

| Allele | Nucleotide Sequence | Amino Acid Sequence |
|---|---|---|
| DQB1.1 | SEQ ID NO: 1 | SEQ ID NO: 2 |
| DQB1.2 | SEQ ID NO: 3 | SEQ ID NO: 4 |
| DQB1.3 | SEQ ID NO: 5 | SEQ ID NO: 6 |
| DQB1.4 | SEQ ID NO: 7 | SEQ ID NO: 8 |
| DQB1.5 | SEQ ID NO: 9 | SEQ ID NO: 10 |
| DQB1.6 | SEQ ID NO: 11 | SEQ ID NO: 12 |
| DQB1.7 | SEQ ID NO: 13 | SEQ ID NO: 14 |
| DQB1.8 | SEQ ID NO: 15 | SEQ ID NO: 16 |
| DQB1.9 | SEQ ID NO: 17 | SEQ ID NO: 18 |
| DQB2 | SEQ ID NO: 19 | SEQ ID NO: 20 |
| DQB3.2 | SEQ ID NO: 21 | SEQ ID NO: 22 |
| DQB3.3 | SEQ ID NO: 23 | SEQ ID NO: 24 |
| DQB3.1 | SEQ ID NO: 25 | SEQ ID NO: 26 |
| DQB4.1 | SEQ ID NO: 27 | SEQ ID NO: 28 |
| DQB4.2 | SEQ ID NO: 29 | SEQ ID NO: 30 |
| DXB | SEQ ID NO: 31 | SEQ ID NO: 32 |

Table 2 shows the amino acid sequence alignments of 15 DQB1 alleles and the sequence of the DQB2 (DXB) locus. The DQB1*0302 alleles provides the consensus sequence, written in one letter code. Sequence homology is indicated by dashed lines, and letters indicate polymorphic residues. The positions for primer annealing, relative to the encoded amino acid sequences shown, are also shown for each primer listed. The serologic DQw type for each allele is also shown, where the type is known. Unnamed alleles and types are indicated by "?".

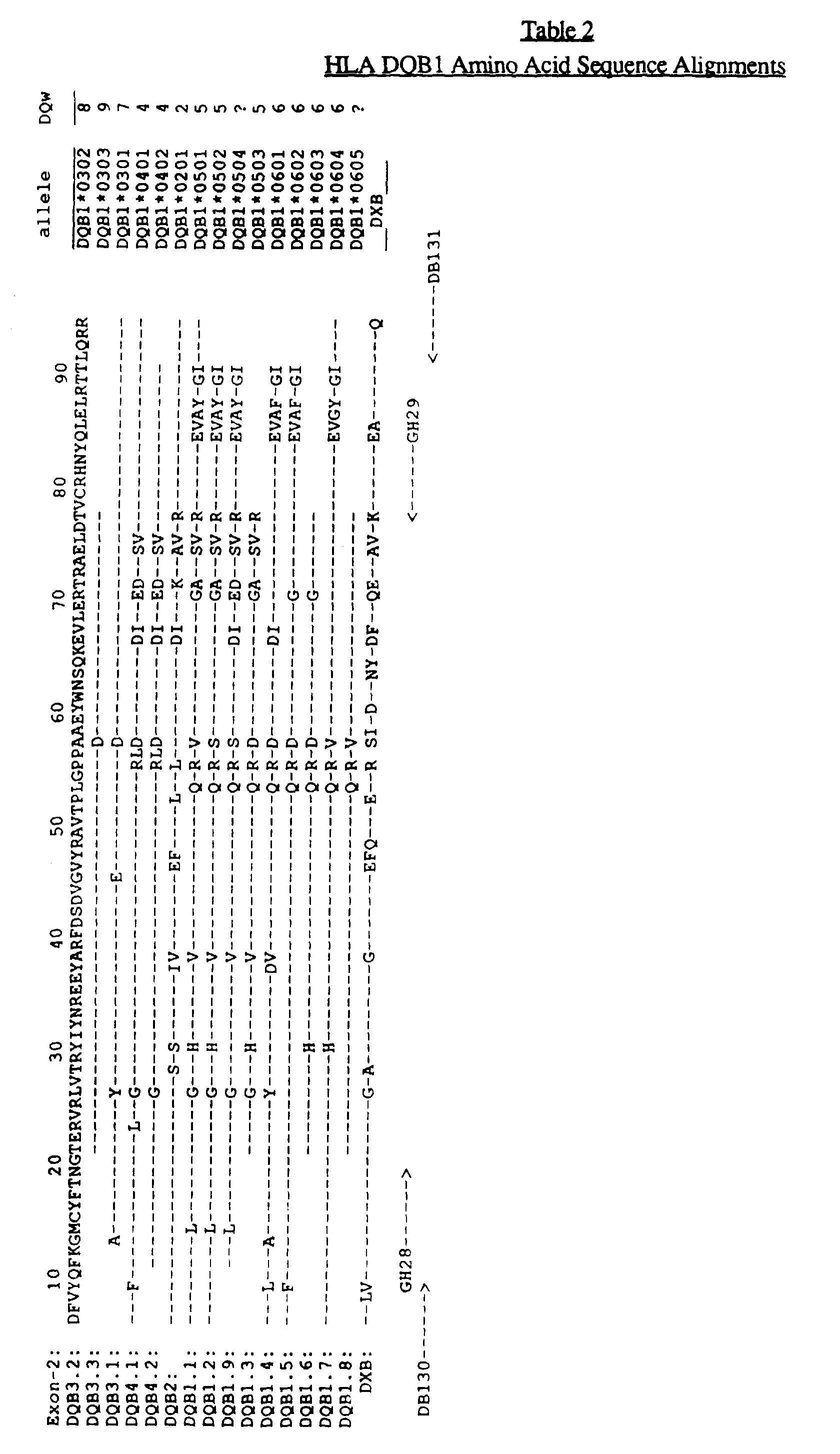

Primers GH28 and GH29, shown in Table 2, have been used for amplifying all HLA DQB alleles (see Horn et al., 1988, supra; Scharf et al., 1988, Proc. Natl. Acad. Sci. USA 85, 3504-3508; EP-A-237,362 and WO 89/04875). However, this primer pair does not amplify the entire second exon of the DQB1 gene, thereby preventing the detection of some polymorphic regions. Furthermore, this primer pair co-amplifies DQB2 allele sequences. The present invention provides "DQB1 specific" PCR primers, which amplify DQB1 alleles but do not amplify the linked pseudogene, DQB2. The present invention also provides "group specific" primers. For example, the group specific primer pair DB130/D886 amplifies the DQB1*02, DQB1*03, and DQB1*04 alleles. Group specific primer pair DB130/UG71 amplifies the DQB1*05 and DQB1*06 alleles.

The DQB1 specific primer pairs DB130/DB131 and DB130/GH29 are specific for DQB1 and do not amplify DQB2. Primer DB130 contains a four base pair mismatch with DQB2. Primer DB131 contains a single base pair mismatch with DQB2 at the second position from the 3' end of the primer, as well as another base pair mismatch with both DQB1 and DQB2 at the third position from the 3' end for the primer. Amplification with the primer pair DB130/DB131 also allows analysis of the polymorphic sequences of the second exon encoding amino acids 78-90. This region lies outside sequences amplified with the primer pair DB130/GH29 primer. However, primer pair DB130/DB131 fails to amplify DQB1.4 (DQB1*0601) efficiently, whereas primer pair DB130/GH29 amplifies all DQB1 alleles, including the DQB1*0601allele, efficiently.

The primer pair DB130/GH29 amplifies all DQB1 alleles efficiently even though a silent polymorphism GTG vs. GTA at codon 78 of the DQB1*04 allele results in the GH29 primer being mismatched, 3 nucleotides away from the 3' end of the primer, when hybridized to a DQB1*04 allele. To ensure efficient amplification of all alleles, the annealing temperature may be lowered, i.e., to 55°C.

Following amplification, SSO probes are used for typing. Control probes are used to determine if DQB1 amplification has occurred. DQB1 "ALL" probe UG86 does not hybridize with DQB2 or DQB1*0401. The sixteen SSO probes of the invention distinguish the 15 DQB1 alleles. Table III shows the sequence of, the allele specificities of, and hybridization and wash conditions for, each of these 16 probes.

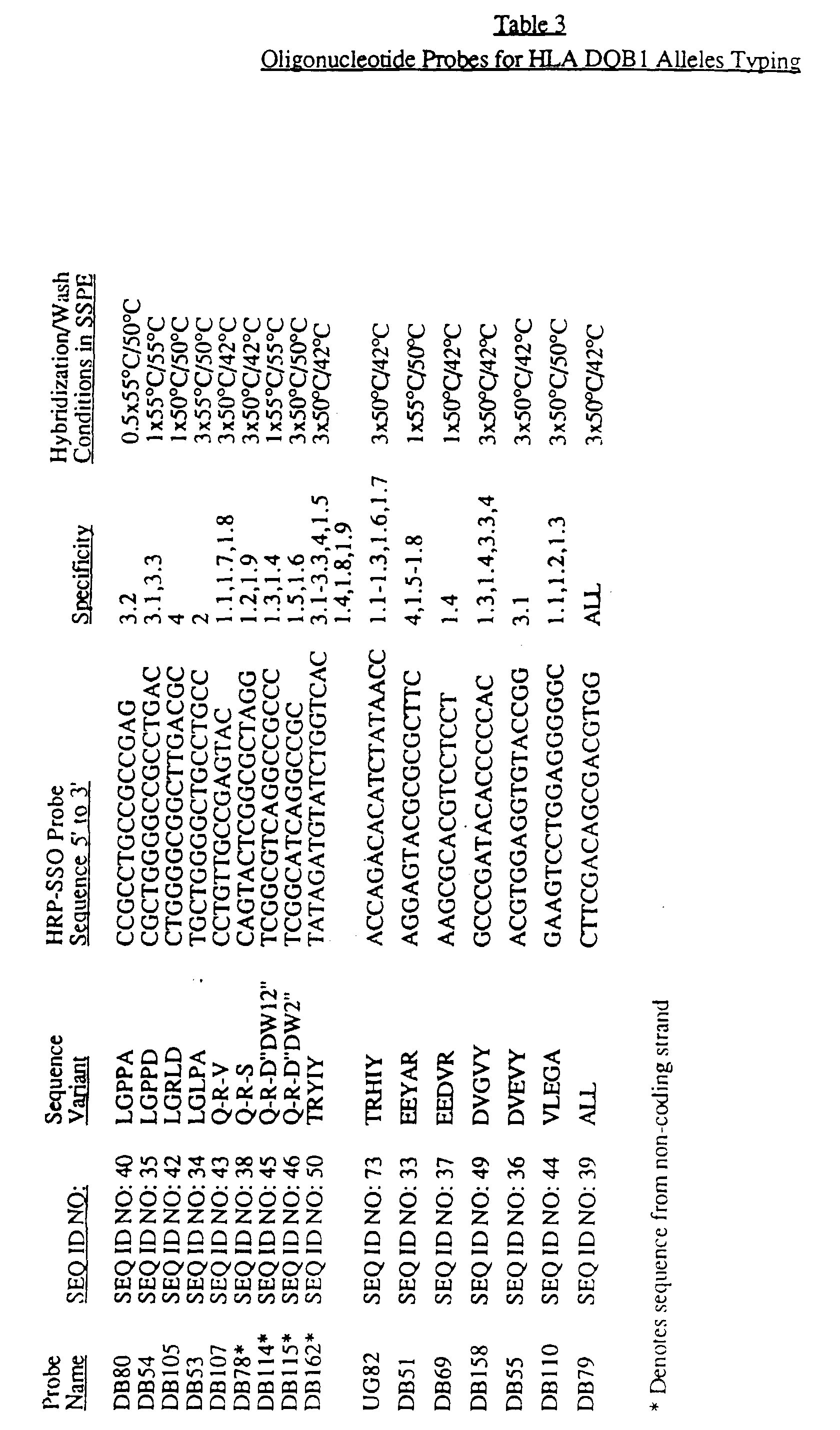

Each probe is used with a hybridization solution that contains 0.5% SDS and SSPE at the concentration and temperature indicated in Table 3, above (for example, the hybridization solution for DB80 contains 0.5X SSPE). The wash is carried out in a solution of 0.1 X SSPE plus 0.1% SDS for 10 minutes at the temperature shown in Table 3, above. Probes DB114 and DB115 distinguish the silent polymorphism, GAC (DQB1.3.1) vs GAT (DQB1.3.2) (Scharf et al., 1989, Proc. Natl. Acad. Sci. USA 86:6215-6219), for the Asp codon at position 57. The probe DB51 also detects a silent polymorphism, GCG (DQB3.1.2) vs GCA (DQB3.1.1), for the Ala codon at position 38, which is useful for distinguishing a homozygous 1.7 or 1.8 individual from a 1.7, 1.8 heterozygous individual.

The sequence-specific oligonucleotide probes of the invention, called "SSOs," when employed in the present methods under the appropriate hybridization and wash conditions, are extremely specific, capable of distinguishing single nucleotide polymorphisms. The SSOs of the invention, unlike the probes used in RFLP methods, can be used to determine not only if alleles are different but also where and how the alleles differ.

The extensive allelic diversity at the HLA DQB1 locus, like that of the other Class II beta genes, is localized primarily to the second exon. In general, the pattern of second exon sequence polymorphism is a patchwork, with specific variants of a polymorphic segments found in a variety of different alleles. In principle, such shared epitopes among different alleles could reflect either common ancestry, gene conversion, or convergent evolution. For purposes of oligonucleotide typing, this patchwork pattern of polymorphism means that many alleles cannot be identified by hybridization to a single oligonucleotide probe, but are identified, instead, by a unique pattern of hybridization with a panel of probes.

Table 4 shows the nucleotide sequence alignments for the HLA DQB1 alleles and the DQB2 (DBX) locus. The local DQB1 allele designations are shown to the left, and to the right are the official WHO HLA nomenclature designations. Probe hybridization sites are also shown in the table; an asterisk (*) indicates that the probe sequence is identical to the noncoding strand. The nucleotide sequence of the DQB1*0302 allele is shown; for the other alleles, only the differences from the DQB1*0302 are shown, and a dash indicates sequence identity.

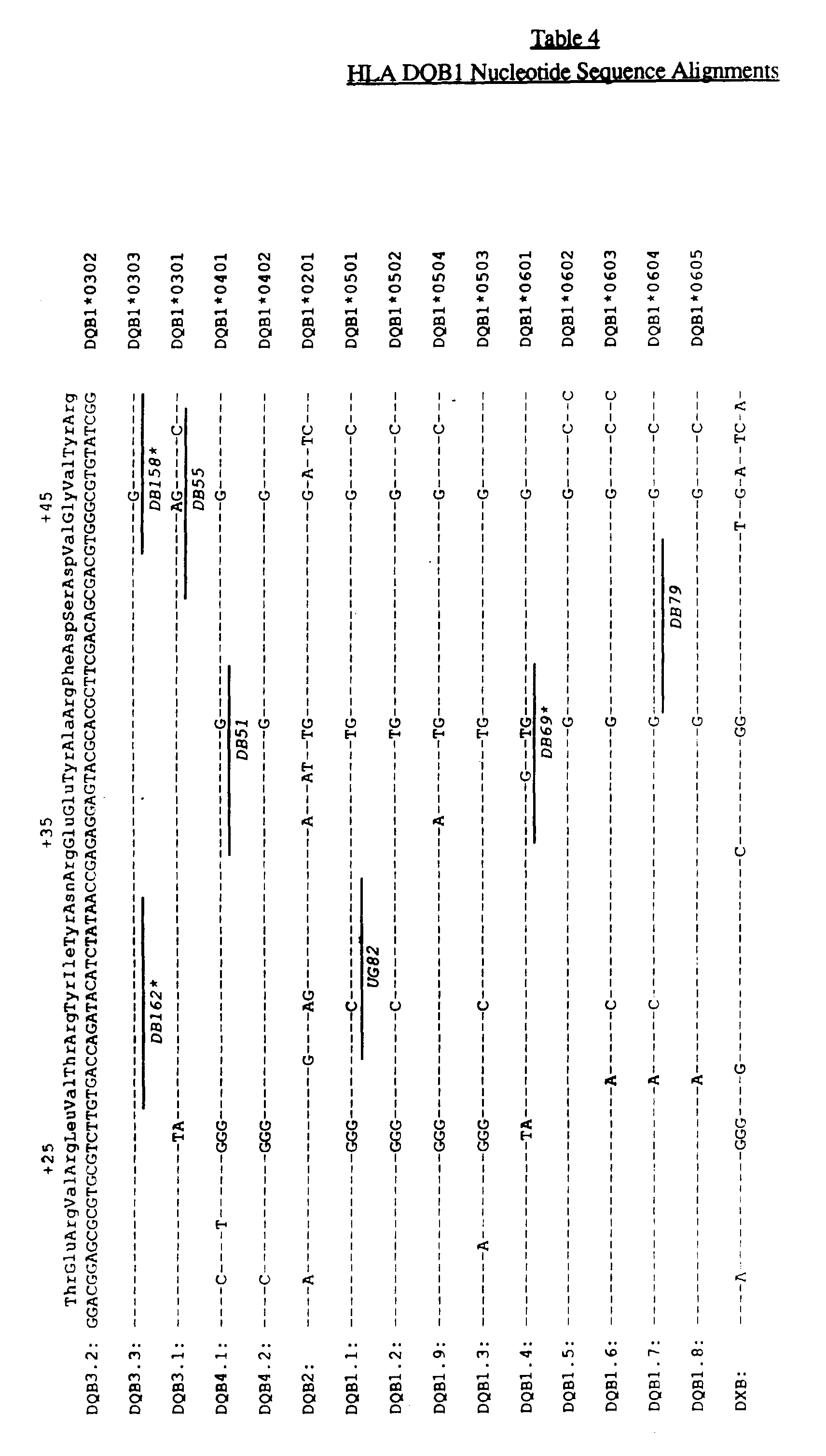

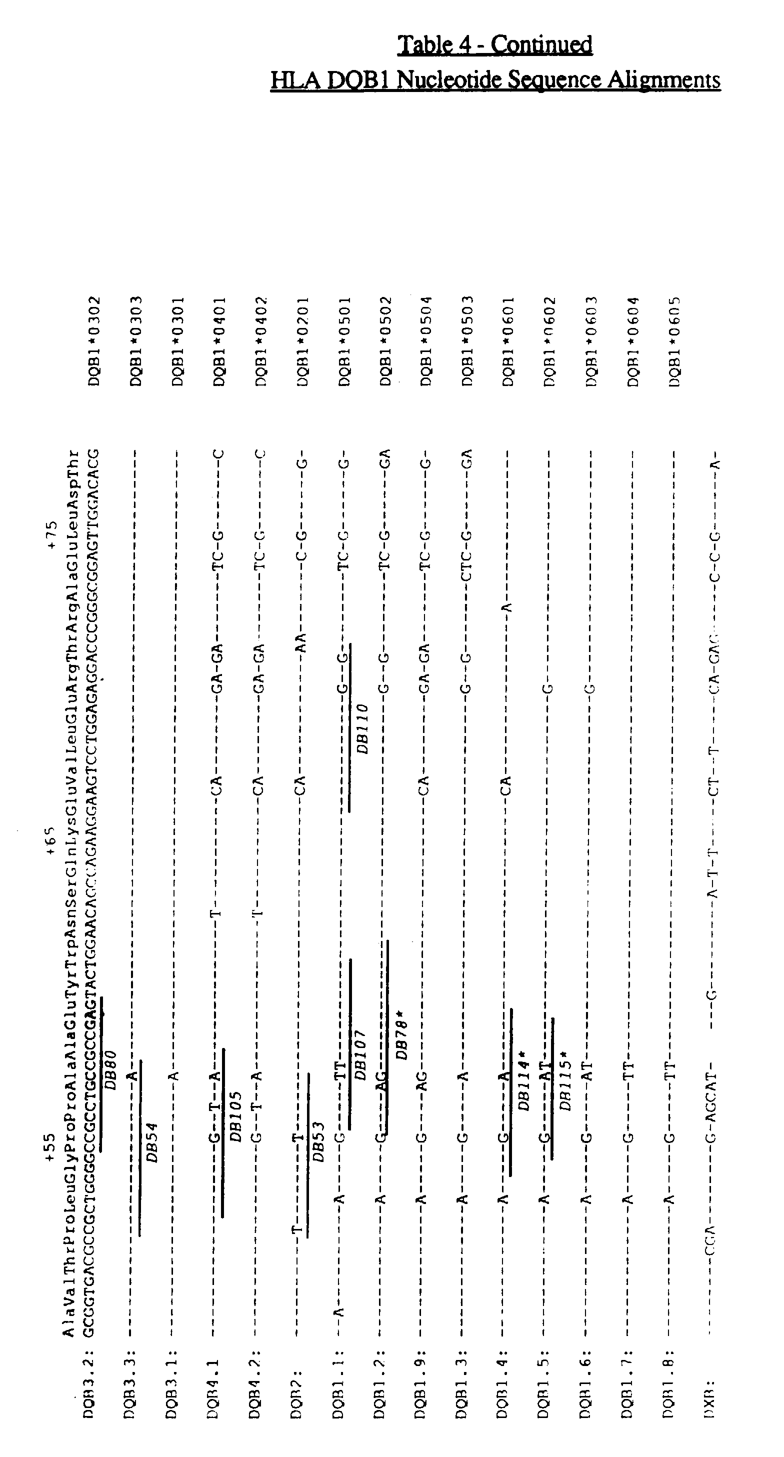

Eight of the sixteen SSO probes used for typing are located in the most variable region around codons 53-57. The other eight probes are located at five other polymorphic regions for the second exon, as depicted above in Table 4. In general, the DQB1 genotype of individual samples is inferred from the hybridization pattern of these sixteen probes. Table 5 shows how the unique patterns of probe hybridization correspond to each of the DQB1 alleles.

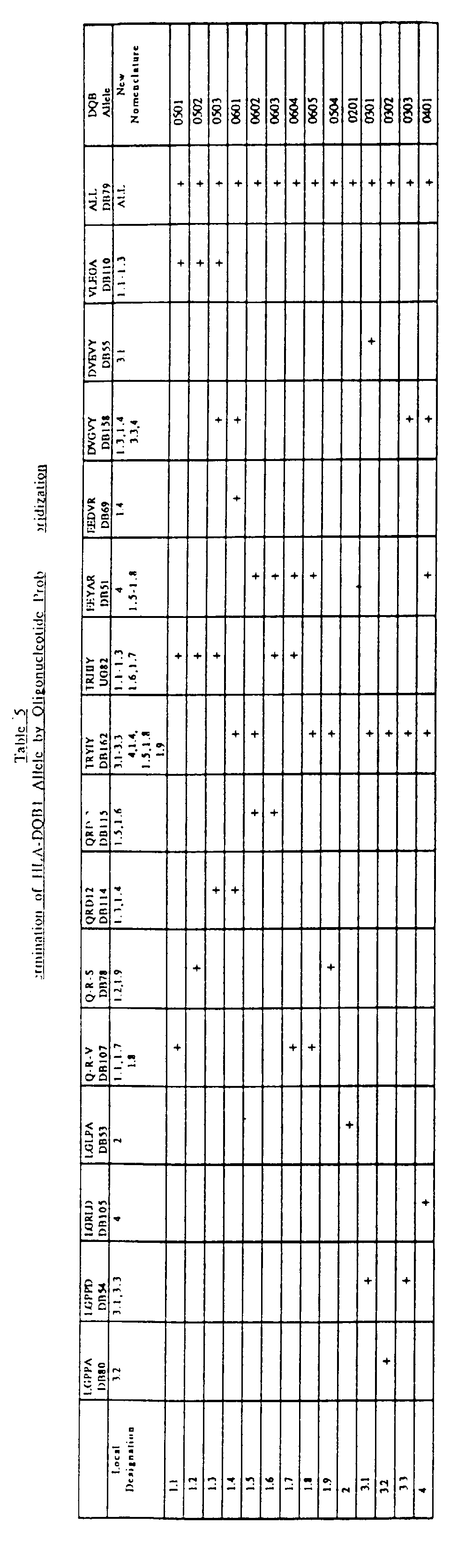

The HRP-SSOs of the invention probes are highly specific under the hybridization and wash conditions shown. Probe DB158, which is required to distinguish homozygous 3.1 samples from heterozygous 3.1, 3.3 samples, however, cross-hybridizes slightly to the 3.2 allele. The examples below illustrate the use of these probes in a method for nonradioactive typing of known homozygous and heterozygous cell lines. The set of 16 probes shown in Table 5 does not distinguish between the following three heterozygous genotypes: 1.5, 1.7; 15, 1.8; and 1.6, 1.8 types. Distinguishing these genotypes requires additional probes.

The HRP-SSOs of the invention allow detection methods that employ chromogenic or chemiluminescent substrates that are easy to use and produce detectable signals rapidly (typically 1 to 10 minutes). The HRP-SSOs are stable for over two years without detectable loss of activity when stored at 4°C. See the article entitled "Nonisotopically labeled probes and primers" by Levenson and Chang, 1989, in PCR Protocols: A Guide to Methods and Applications (Innis, Gelfand, Sninsky and White ed., Academic Press, Inc. San Diego). Radiolabelled probes can be employed but are not necessary for excellent sensitivity, an important benefit provided by the present invention.

The dot blot format for detection enables the rapid typing of a large number of samples and will be useful in determining the allele frequencies of HLA DQB1. A recently developed alternative for PCR/SSO DQbeta typing is the immobilized dot blot format. See the article entitled "Genetic analysis of amplified DNA with immobilized sequence-specific oligonucleotide probes" by Saiki et al., 1989, Proc. Natl. Acad. Sci. USA 86:6230-6234, and copending Serial No. 347,495, filed November 30, 1989. In this procedure, the SSO probes are applied and fixed to the filter (rather than the amplified DNA), hence the term "reverse dot blot".

The reverse dot blot procedure allows a single sample to be analyzed in a single hybridization with a membrane containing an array of immobilized probes. The conventional dot blot format is useful when the number of samples exceeds the number of probes used (e.g., patient versus control or population genetics studies). The reverse dot blot format is valuable for clinical, diagnostic, and forensic analyses, where there are fewer samples than probes.

The panel of probes disclosed herein is capable not only of distinguishing the 15 DQB1 alleles currently known, but also of detecting new alleles, revealed by novel patterns of probe hybridization. The new DQB1 allele 1.9 was initially identified by an unusual pattern of SSO probe hybridization in samples from an IDDM patient and his mother and then confirmed by cloning and sequencing the PCR product from these samples (see Example 5). This allele appears to be a "recombinant" allele, sharing most of the second exon sequence with DQB1*0502 and the third hypervariable region with DQB1*04.

The present invention is suitable for commercialization as a kit for HLA DQB1 typing. The kit can contain probes for typing the DQB1 alleles. Such a kit may also include reagents for PCR or other amplification procedures; such reagents include oligonucleotide primers, a thermostable DNA polymerase, and nucleoside triphosphates.

The following examples show illustrative embodiments of the present invention. The examples show that the present invention provides, in a preferred embodiment, a nonisotopic PCR/SSO system for HLA DQB1 typing that is simple, rapid, and capable of precise DQB1 typing for a variety of samples from different sources.

### Example 1

### Optimization of HLA DQB Amplification Conditions

Human genomic DNA, 0.5-1.0 µg, was amplified as described in the article entitled "Primer-Directed Enzymatic Amplification of DNA with a Thermostable DNA Polymerase" by Saiki et al., 1988, Science 239:487-491, and in Scharf et al., 1988, Hum. Immunol. 22:61-69, and Scharf et al., 1989, Proc. Natl. Acad. Sci. USA 86:6215-6219. Sample DNA was recovered from cell lines LG2 and LUV and amplified in a 100 µl reaction with 2.5 units of Taq polymerase. For each primer set and MgCl₂ concentration, three different temperature profiles for PCR were investigated. All amplification reactions were carried out using a PECI Thermal Cycler (Perkin Elmer Cetus Instruments, Norwalk, CT) and Taq polymerase.

The samples were amplified for 35 cycles. The three-step PCR profile involved denaturing the DNA at 94°C for one minute, annealing the primers at 55°C for 30 seconds, and extending the primers with Taq polymerase at 72°C for 30 seconds. The two-step PCR profile involved denaturing the DNA at 94°C for 1 minute and annealing and extending the primers at either 60°C or 65°C for 40 seconds. A negative control (no DNA) was always included to check for contamination. Three µl of amplified product were loaded in a 3% Nusieve, 1% Agarose gel to monitor the amplification efficiency. Primers GH28, GH29, DB130, and DB321 were tested in various combinations.

The samples amplified with 60°C annealing and extending steps gave more specific product with little or no background of the other samples, and, in all cases, the reaction buffer containing 1.5 mM MgCl₂ gave a more specific and efficient amplification than the reaction buffer containing 2.5 mM MgCl₂.

### Example 2

### Coamplification of DQB1 and DQB2

To determine if the primer pairs and reaction conditions described in Example 1 coamplified DQB1 and DQB2, the same reactions were repeated using DNA from the LUY cell line. After amplification, a small portion of the amplified DNA was denatured and applied to a series of nylon filters. These dot blots were then hybridized with DQB2 and DQB1 "ALL" probes as follows. Each filter was incubated in hybridization buffer with one of the labelled probes. Each SSO probe was covalently conjugated to horseradish peroxidase (HRP) for nonisotopic detection.

To prepare the dot blots, about 5 µl of each amplified DNA sample were mixed with 95 µl of a denaturation solution composed of 0.4 M NaOH and 25 mM EDTA, and the resulting mixture was applied to pre-wetted (water or 2 X SSPE) nylon filters (BioDyne B, Pall Corp., Glen Cover, NY, or Genatran 45, Plasco; Woburn, MA) using a dot blot manifold (BioRad, Richmond, CA). Duplicate membranes were prepared. The DNA was immobilized on the nylon filter by ultraviolet irradiation at a flux of 50 mJ/cm² with a Stratalinker™ (Stratagene, La Jolla, CA) UV light box.

Membrane A was incubated in hybridization buffer with DQB2 probe GH63 (SEQ ID NO: 71; 5'-CTCGATGCTCCGCCCCAG-3') and membrane B with HRP-labelled DQB1 "ALL" probe UG86 (SEQ ID NO: 74; 5'-TACTGGAACAGCCAGAAGGA-3'). Hybridization was carried out at 50°C in 3 X SSPE, 0.5% SDS for 30 minutes, and the filters were then washed with 0.1 X SSPE, 0.1% SDS for 10 minutes in a 42°C water bath. The probe UG86 does not hybridize with DQB2 or DQB1*0401 alleles. Probe GH63 (non-coding sequence) hybridizes only with DQB2.

The presence of hybridized probe was detected either by a chemiluminescent substrate (ECL; Amersham, Arlington Heights, IL) or by the chromogenic dye substrate TMB (3,3', 5'5-tetramethylbenzidine [Fluka; Ronkonkoma, NY]), which is converted to a blue precipitate by HRP in the presence of hydrogen peroxide. All incubations were at room temperature with moderate shaking. For chemiluminescent detection, a stringent wash was completed, and the membranes were incubated for 30 minutes in 1 X Dulbecco's PBS and then placed in the ECL detection kit for 1 minute and exposed to X-ray film for 1-5 minutes.

For detection with TMB, the membranes were rinsed in Buffer C (100 mM sodium citrate, pH 5) for 5 minutes and then incubated in Buffer C containing 0.1 mg/ml TMB (stock is 2 mg/ml in ethanol) plus 0.00015% H₂O₂ for 2-10 minutes. The reaction was stopped by rinsing the membranes in 0.01 X Buffer C; membranes were then photographed for a permanent record. The reagents for TMB-based detection of HRP-labeled oligonucleotides are commercially available from Perkin-Elmer Cetus Instruments; detailed protocols for their use are available in the AmpliType™ DQα DNA Typing Kit product insert.

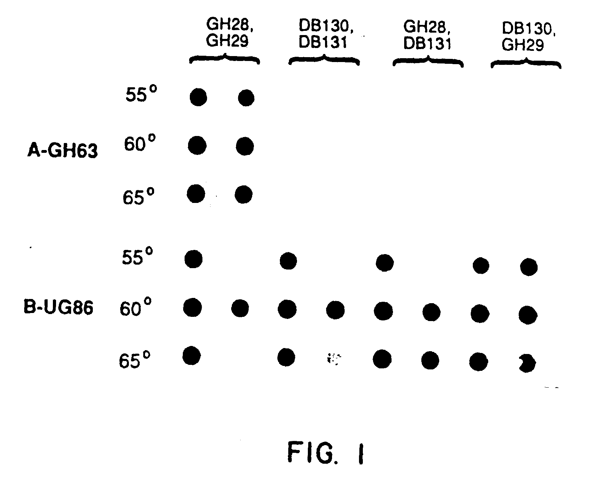

As shown in Figure 1, the dot blot results demonstrate that samples 1 and 2, which were amplified with primers GH28 and GH29, hybridized with both probes equally, suggesting both loci were amplified to about the same degree. Samples amplified with DB130 and DB131 at three different annealing temperatures did not hybridize with the DQB2 probe, while samples amplified with GH28 and DB131 or with DB130 and GH29 hybridized very faintly with the DQB2 probe.

### Example 3

### HLA DQB1 Amplification

The PCR amplification and dot blot results of Examples 1 and 2 show that the DB130/DB131 primer pair is specific for DQB1, does not amplify DQB2, and so is a preferred pair for general DQ typing. In further testing, DNA samples from homozygous typing cell (HTC) lines were amplified as described in Example 1. Interestingly, the DQB1.4 (DQB1*0601) allele failed to amplify with the DB130/DB131 primer pair at 65°C; however, when the annealing temperature was lowered to 60°C or 55°C, the allele was amplified, although somewhat inefficiently. The primer pair DB130/GH29 specifically and efficiently amplified all known DQB1 alleles, in spite of the mismatch resulting from the silent polymorphism (GTG vs. GTA) at codon 78 in the hybridization region for the GH29 primer. DQB1*0402 alleles are mismatched (A-A) three nucleotides from the 3' end of GH29, as determined by amplification with DB130 and DB131. Nonetheless, DQB1*0402 alleles from the HTCs ARC, OLN, and RSH, as well as heterozygous samples that have the same codon 78 (GTA), do amplify with the primer pair DB130/GH29, showing that this mismatch does not prevent amplification. Thus, the primer pair DB130/GH29 has the requisite "locus specificity" and "allelic range" to be suitable for routine DQB1 typing.

### Example 4

### HLA DQB1 Typing

Previously sequenced homozygous and heterozygous cell lines were used to demonstrate the suitability of the sixteen SSO probe system for HLA DQB1 typing. Genomic DNA from 10 different cell lines was amplified as described above with primers DB130 and GH29, or with primers DB130 and DB131, for 35 cycles using the following temperature profile: 94°C template denaturating for 1 minute and either 55°C or 60°C primer annealing and extending for 40 seconds. Five µl of PCR amplified product were spotted onto a membrane following amplification. Enough amplified DNA and denaturing solution were prepared to make 16 replicate membranes. Generally, if amplified DNA is limiting, membranes can be decolorized in 0.5% sodium sulfite at room temperature with shaking for 10-20 minutes, and the hybridized probes removed by incubating the strips at 70°C in 0.1 X SSPE, 0.1% SDS for 1 hour. The membranes can then be re-hybridized.

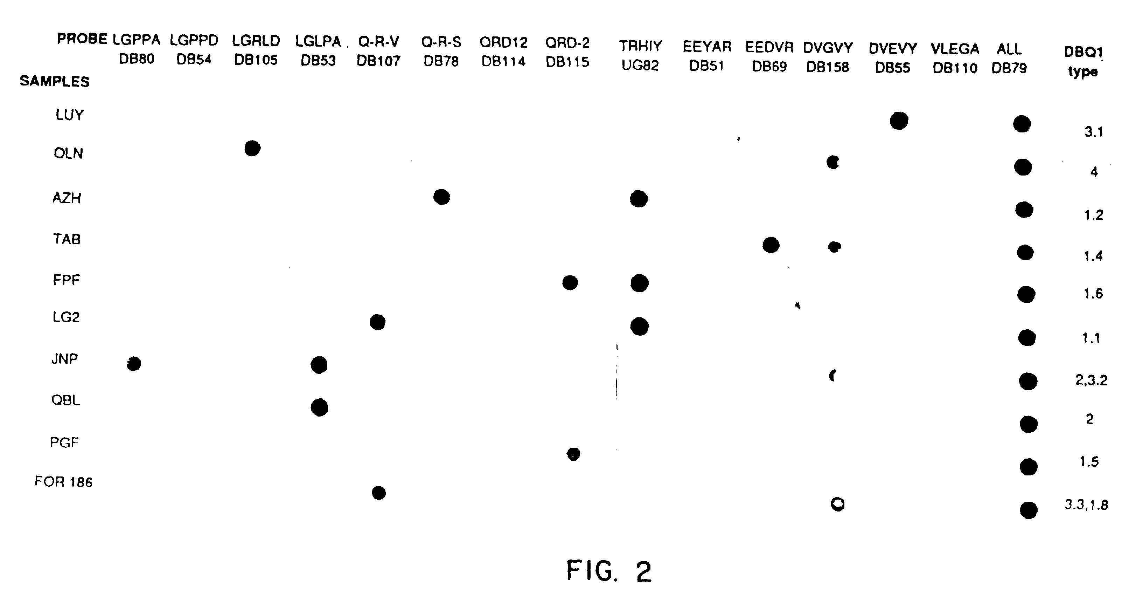

Membranes were hybridized to each of the 16 HRP-labeled probes described in Table 3 for 30 minutes to 1 hour with 1.5 pmole of probe per ml of hybridization solution. Table 3 shows the sequence, hybridization, and wash conditions for each probe. Figure 2 shows the results of the DQB1 typing on known homozygous and heterozygous cell lines. In the Figure, the probe names as well as the amino acid sequences encoded in the probe region are indicated on the right; the sample names are written on top.

All of the HRP-labelled probes are highly specific under the hybridization and wash conditions used here, except probe DB158, which distinguishes homozygous 3.1 samples from heterozygous 3.1, 3.3 samples. This probe cross-hybridizes slightly to the 3.2 allele.

Table 6 shows the assignments of the DQB1 genotypes based on the probe reactivity for the 10 different samples, two of which were heterozygous cell lines. The local designation is used to denote probe specificity. The HLA committee nomenclature is shown to the right. A computer algorithm has been designed to give the DQB1 genotype by entering the pattern of probe hybridization obtained for a given sample. Such a program was used to calculate that only three of the 120 possible genotypes are not resolved by this panel of probes. The three genotypes not resolved by this panel of probes are the heterozygous genotypes, 1.5, 1.7; 15, 1.8: and 1.6, 1.8.

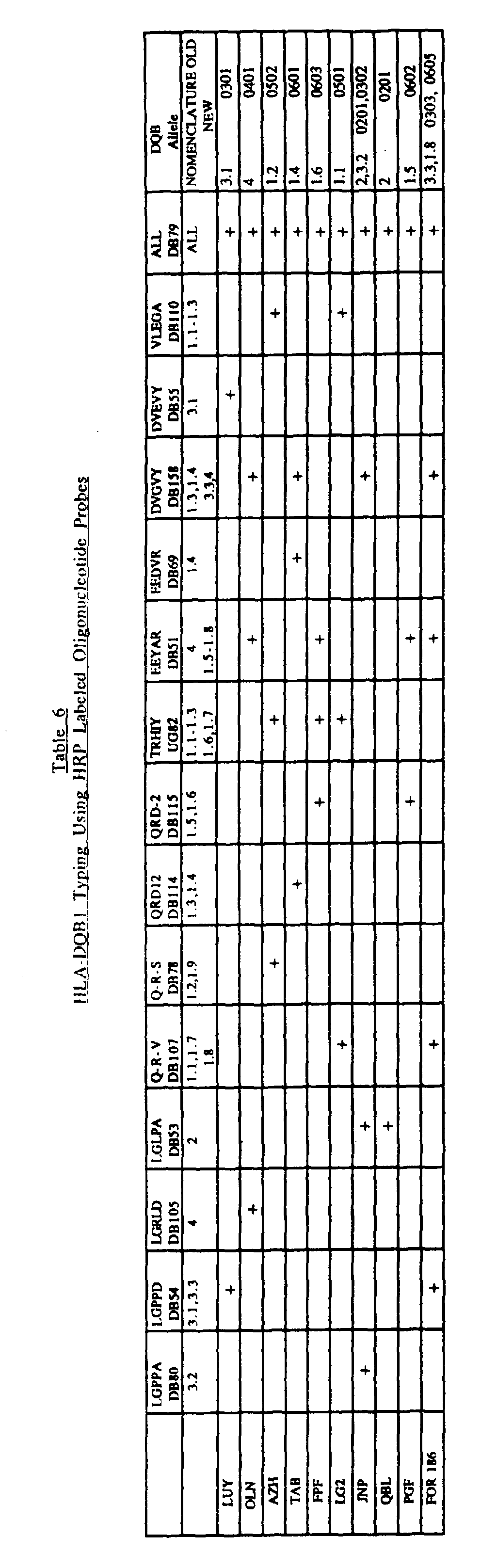

### Example 5

### Identification of a Novel HLA DQB1 Allele

A highly unusual family has been identified in which two HLA identical siblings (DR1/DR2) have IDDM (Eisenbarth et al., 1985, Diabetes Care 8:477-480), and a third (also DR1/DR2) has recently developed IDDM. In this family, the three siblings who shared the maternal DR1 and the paternal DR2 haplotype all have IDDM as well as islet cell antibodies. This striking pattern suggested that these two haplotypes were predisposed to IDDM. To identify susceptibility alleles in this family, the sequences of the polymorphic second exon of the DQB1, DRB1, and DRB5 loci on the two predisposing haplotypes were determined (see Erlich et al., 1991, Diabetes, supra).

Samples of genomic DNA from a sibling and the parents were obtained. Polymerase chain reaction amplification and oligonucleotide typing for the HLA DQA1 loci were performed as described in the instruction manual, incorporated herein by reference, for the AmpliType™ DQα DNA typing kit, marketed by PECI. Reactions, in a volume of 100 µl, contained standard PCR salts, 200 µM of each dNTP, 1 µg of genomic DNA, and 2.5 units of Taq polymerase (PECI, Norwalk, CT, AmpliTaq™ DNA polymerase) and were performed on a PECI Thermal Cycler programmed for 35 cycles of: 95°C denaturing for one minute, 55°C annealing for 30 seconds, and 72°C extending for 45 seconds.

PCR product was subsequently digested with BamHI and PstI restriction endonucleases (Boehringer-Mannheim), purified by extraction with phenol:chloroform and Centricon 30 (Amicon) dialysis, and ligated into M13mp18 with T4 DNA ligase (BRL). E. coli DG98 was transformed with the resulting ligated DNA, and the transformants were used to prepare plaques, which were probed with nick-translated cDNA probes for the appropriate locus. Positive plaques were used to infect a plate of DG98, and confluent plates were extracted the following morning by gentle shaking in Luria broth (5 ml). Template DNA was prepared for each clone of interest and then sequenced. Multiple PCRs, clonings, and sequencing reactions were done to confirm the new sequence, and to ensure that a potential misincorporation had not occurred or that misprimed shuffling (Saiki et al., 1988, Science 239:487-491) capable of generating an artifactual "hybrid" sequence was not involved. DNA sequencing was carried out with the dideoxy chain termination process (Sanger et al., 1977, Proc. Natl. Acad. Sci. USA 74:5463-5467). DQB1 DNA sequences were confirmed by oligonucleotide typing.

DNA samples from a DR1/DR2 sibling, the mother, and the father, were analyzed by PCR amplification of the DQalpha, DQB1, and the DRB loci; the amplified DNA was characterized by M13 cloning and dideoxy chain termination sequencing and by dot blot hybridization with SSO probes (Erlich and Bugawan, pp. 193-298, PCR Technology: Principles and Applications for DNA Amplification, Erlich ed., 1989, Stockton Press, New York; and Saiki, 1986, supra).

The serology and DNA typing of this family is shown below.

| Haplotype Designation | Serology | DNA Type |
|---|---|---|
| A | A3, B7, DR2 | DQA1*0401, DQB1*0402, DRB1*1501 |
| B | A11, Bw35, DR4 | DQA1*0301, DQB1*0301, DRB*04₋₋ |
| C | A26, Bw49, DR1 | DQA1*0102, DQB1*0504, DRB1*0101 |
| D | A25, B18, DR2 | DQA1*0102, DQB1*0602, DRB1*1501 |

| Individual | Haplotype |
|---|---|
| Father | A/B |
| Mother | C/D |
| IDDM Male Sibling | A/C |
| IDDM Male Sibling | A/C |
| IDDM Male Sibling | A/C |
| non-IDDM Male Sibling | B/D |
| non-IDDM Female Sibling | A/D |
| non-IDDM Female Sibling | B/C |

The DR2 haplotype in the IDDM sibling contains a conventional DRB1 allele (Dw2 or *1501) common to most Caucasian DR2 haplotypes (WHO Nomenclature Committee, 1990, Immunogenics 31:131-140, and Marsh and Bodmer, 1989, Immunol. Today 10:305-312), This haplotype typically contains the DQA1 allele 1.2 or *0102 and the DQB1 allele 1.5 or *0602, as does the maternal DR2 haplotype. In this family, however, the paternal DR2 haplotype contains the DQA1 allele 4.2 or *0401 and the DQB1 allele 4.2 or *0402. This combination of DQA1 and DQB1 alleles is usually found only on DR8 haplotypes in Caucasian and is quite rare (<4%). The DRB1 allele of the DR1 haplotype also contains the conventional DR1 sequence *0101; DR1 haplotypes typically contain the DQA1 allele *0101 and the DQB1 allele *0501. This DR1 haplotype, however, contains the DQA1 allele *0102, and a novel, previously unreported DQB1 allele, termed provisionally DQβ1*05new, which appears to be a "recombinant" between the DQB1*0502 and the *0402 alleles. The DQβ1*05new allele has been renamed DQB1*0504 (DQB1.9), as in Tables 2 and 4, above.

This interpretation is consistent with the premise that the highest IDDM susceptibility is associated with the presence of two predisposing haplotypes. The mother (age 55), who possesses the unusual DR1 haplotype along with the conventional DR2 haplotype, has islet cell antibodies (ICA) but does not have IDDM and has maintained normal first-phase insulin secretion on prospective evaluation.

The DR2, Dw2 haplotype is strongly negatively associated with IDDM and therefore has been considered "protective" while the DR1 haplotype is weakly associated with IDDM (Thompson, 1988, Ann. Rev. Genet. 22:31-50), Given the strong linkage disequilibrium between the DQ and DR regions of HLA class II haplotypes, it has been difficult to attribute these haplotype disease associates to specific class II alleles. The analysis of highly predisposing haplotypes with an unusual combination of DQB1 and DRB1 alleles, like those observed in the family described above, allows the tentative identification of predisposing alleles. On the DR2 haplotype, the DQB1*0402 allele is implicated in susceptibility because the DRB1 allele (*1501) is the same as the "protective" DR2, Dw2 haplotype. Thus, it is the conventional DQB1 allele *0602 on DR2, Dw2 haplotypes that is likely responsible for the "protection" associated with this haplotype. The DQB1 allele found on this unusual DR2 haplotype is identical to the DQB1*0402 allele found on Caucasian DR8 haplotypes. DR8 is weakly associated with IDDM (Thompson, supra). A similar DQB1 allele, *0401, is also found on Japanese DR4 haplotypes, which are positively associated with IDDM (Aparico et al., 1988, Immunogenetics 28:240-246) but in this population, it is linked to the DQA1*0301 allele.

Studies of HLA class II sequence polymorphism and IDDM have revealed a general correlation with the charge of the amino acid residue at position 57 of the DQB1 chain (Morel et al., 1988, Proc. Natl. Acad. Sci. 85:8111-8115). One recent study reported that the presence of an Asp at position 57 was, per se, "protective." Because the DQB1*0402 allele found on this DR2 haplotype has an Asp residue at position 57, the results described in Example 5 indicate that Asp 57 is not absolutely protective. Of the six DR2 haplotypes found in the four DR2+ IDDM patients studied, five patients have Asp 57, as shown in Table 7.

**Table 7**

| DQB1 Alleles on Six DR2 Haplotypes in Four Unrelated DR2+ IDDM Patients | | | |
|---|---|---|---|
| Number | Allele | Codon 57 Amino Acid | DR Type |
| 2 | DQB1*0602 | ASP | DR2/DR- |
| 1 | DQB1*0502 | SER | DR2/DR3 |
| 2 | DQB1*0603 | ASP | DR2/DR- |
| 1 | DQB1*0402 | ASP | DR2/DR1 |

Only two patients have the DQB1*0602 allele, which is expected in >90% of Caucasian DR2 haplotypes. Possibly the entire allele DQB1*0602 confers resistance, rather than a single residue at position 57 of the DQB1 chain.

The methods described herein led to the identification of a new DQB1 allele. DQβ1*05new (also named DQB1.9 and DQB1*0504), found on the unusual DR1 haplotype. This allele may also be responsible for conferring IDDM susceptibility because the DRB1 allele (*0101) observed in the patient is the one typically found on control DR1 haplotypes. This novel DQB1 allele has Ser at position 57 like the rare DQB1 allele (*0502) previously identified in a DR2+ IDDM patient (Horn, 1988, supra., and Erlich et al., 1990, Diabetes 39:96-103). The new allele appears to be a complex "recombinant" of the *0502 and the *0402 alleles; this sequence may have been created by gene conversion due to the insertion of a DQB1*0402 segment into the DQB1*0502 allelic framework, with the sites of a putative recombination somewhere between codons 58 and 74. According to the structural model for HLA Class II molecules, a peptide binding groove is formed by a β-pleated sheet and two α-helices; thus, in this new allele, a segment encoding part of the α-helix is common to *0402 while the rest of the DQB1 chain is similar to the DQB1*0502 allele.

### Example 6

### DQB1 Typing - Reverse Dot Blot Format

In this embodiment of the invention, the DQB1 probes are fixed to a membrane, and the amplified target DNA is hybridized to the membrane-bound probe. The set of typing probes is designed so that each probe will hybridize to a specific target sequence at the same temperature and salt concentration (and stay hybridized under the same wash conditions) as all other probes in the set. The PCR primers used in the amplification are biotinylated, as described in the book PCR Protocols (1990, Academic Press, San Diego, CA), so that any amplified DNA that hybridizes to the membrane-bound probes can be easily detected.

In one embodiment, detection is carried out by reacting streptavidin (SA)-conjugated horseradish peroxidase with any biotinylated, amplified DNA hybridized to the membrane-bound probe. The HRP thus becomes bound, through the SA-biotin interaction, to the amplified DNA and can be used to generate a signal by a variety of well know means, such as the generation of a colored compound, e.g., by the oxidation of tetramethylbenzidine (see U.S. Patent No. 4,789,630).

Although the probes can be fixed to the membrane by any means, a preferred method involves "tailing" an oligonucleotide probe about 13 to 25 nucleotides in length with a much longer sequence of poly-dT. The resulting poly-dT "tail" can then be reacted with amine groups on the membrane to fix the probe covalently to the membrane. This reaction can be facilitated by UV irradiation.

Terminal deoxyribonucleotidyl transferase (TdT, Ratliff Biochemicals; for the reactions below assume a concentration of about 120 Units/µl, which is 100 pmol/µl) can be used to create a poly-dT tail on a probe, although one can also synthesize the tailed probe on a commercially available DNA synthesizer. When one uses a DNA synthesizer to make the tailed probe, however, one should place the tail on the 5' end of the probe, so that undesired premature chain termination occurs primarily in the tail region.

TdT reactions should be carried out in volume of about 100 µl containing 1X TdT salts, 200 pmol of oligonucleotide, 800 µM dTT, and 60 units of TdT. 10X TdT salts is 1,000 mM K-cacodylate, 10 mM CoCl₂, 2 mM dithiothreitol, 250 mM Tris-Cl, pH 7.6, and is prepared as described by Roychoudhury and Wu, Meth. Enzymol. 65:43-62. A 10X stock solution of 8 mM dTTP can be prepared (neutralized to pH 7 with NaOH) for convenience.

The TdT reaction should be carried out at 37°C for two hours and then stopped by the addition of 100 µl of 10 mM EDTA, pH 8. The final concentration of tailed oligonucleotide is 1 µM (1 pmol/µl), and the length of the homopolymer tail is about 400 residues. Tail length can be changed by adjusting the molar ratio of dTTP to oligonucleotide. The tailed probes can be stored at -20°C until use.

Two types of nylon membrane are preferred for the reverse dot blot format: Biodyne™ nylon membrane, 0.45 micron pore size, manufactured by Pall; and Biotrans™ nylon membrane, 0.45 micron pore size, manufactured by ICN. The probes can be spotted onto the membrane very conveniently with the Bio-Dot™ dot blot apparatus manufactured by BioRad. Each probe is spotted onto a unique, discrete location onto the membrane. About 5 to 10 picomoles of each tailed probe is premixed with 60-100 µl of TE buffer before application to the dot blot apparatus. After dot blotting, the membrane is briefly placed on absorbent paper to draw off excess liquid.

The membrane is then placed inside a UV light box, such as the Stratalinker™ light box manufactured by Stratagene, and exposed to 50 to 60 millijoules of flux to fix the tailed probe to the nylon membrane. After a brief rinse (for about 15 minutes in hybridization solution) to remove unbound probe, the membrane is then ready for hybridization with biotinylated PCR product. One-half to one picomole (one-quarter to one-half of a typical, 100 µl PCR mixture) of PCR product is added to each probe panel for hybridization. About 50 µl of streptavidin-horseradish peroxidase (SA-HRP, commercially available from PECI; see the instruction manual for the AmpliType™ DQα DNA Typing Kit) conjugate can be added at this time for convenience, but better signals will result if a separate SA-HRP incubation and wash, at room temperature, is performed after the stringency wash.

Hybridization is typically carried out at 50°C for 30 minutes in a water bath and with hybridization buffer composed of 0.5% SDS and 3X to 5X SSPE, most commonly 4X. Stringency washing is carried out at 50°C for 15 minutes in a water bath and with wash solution composed of 0.1% SDS and 1X SSPE. A post-wash of 1X PBS at room temperature for 30 minutes can enhance signal quality.

The hybridizing regions of the tailed probes for use in the reverse dot blot method are shown below. X is inosine. Also shown are the target sequence, given in one letter amino acid code, and allele specificity of each probe.

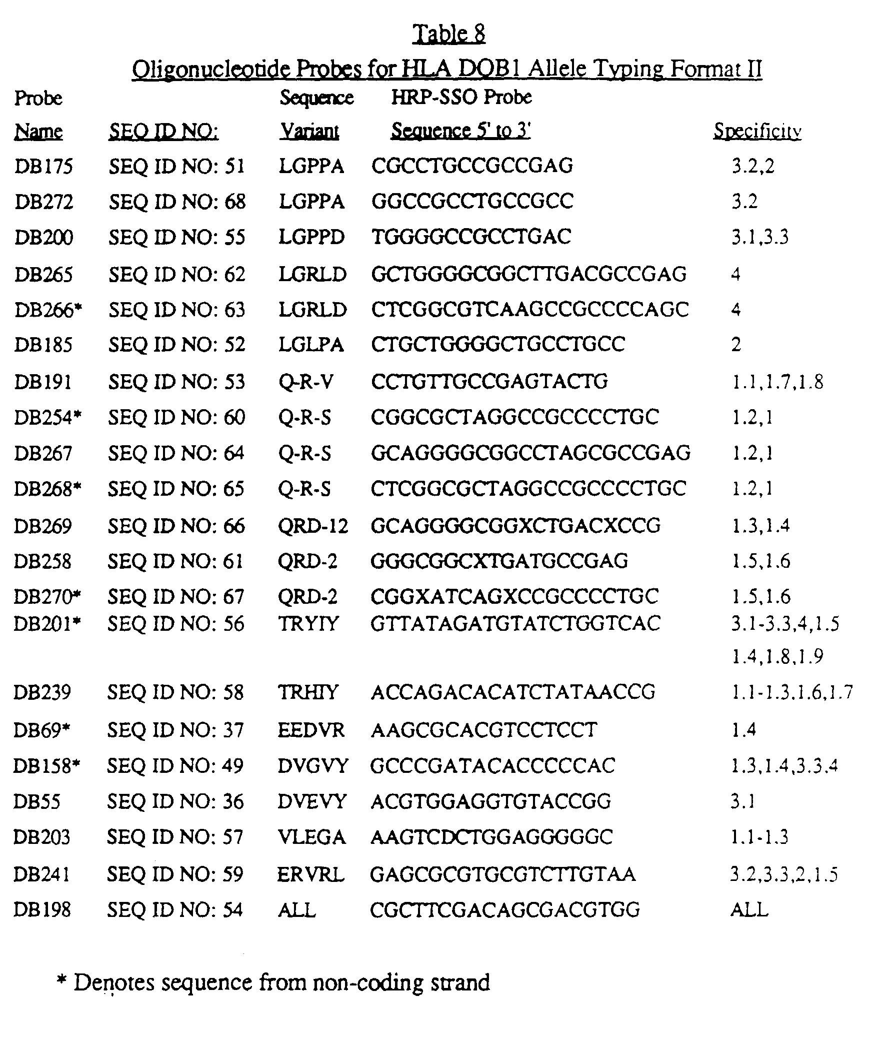

## Claims

1. A method for determining the DQB1 type of nucleic acids in a sample, which method comprises (a) amplifying DQB1 gene second exon sequences without amplifying alleles of closely related genes such as DQB2 (DXB) or the DPB1 and DRB genes in the presence of a primer specific for all of the alleles of the DQB1 gene; (b) hybridizing any amplified nucleic acid with a set of oligonucleotide probes under conditions such that only probes hybridized to exactly complementary sequences can remain stably bound to the amplified DNA; and (c) determining whether amplification has occurred.

2. The method of claim 1, wherein the primer used for the amplification is selected from the group consisting of DB130 (SEQ ID NO:47) and DB131 (SEQ ID NO:48), preferably the primer pair DB130 (SEQ ID NO:47)/DB131 (SEQ ID NO:48) or the primer pair DB130 (SEQ ID NO:47) and GH29 (SEQ ID NO:70).

3. The method of claim 1 or 2, wherein said amplifying step is accomplished by PCR.

4. The method of any one of claims 1 to 3, wherein the set of oligonucleotide probes comprises two or more probes selected from the group consisting of DB51, DB53, DB54, DB55, DB69, DB78, DB79, DB80, DB105, DB107, DB110, DB114, DB115, DB158, DB162, and UG82.

5. The method of claim 4, wherein said probes are labeled.

6. The method of claim 4 or claim 5, wherein said set of probes is immobilized on a solid support, and each different probe is located at a distinct and discrete location on said solid support.

7. A pair of oligonucleotide primers specific for all of the alleles of the DQB1 gene, which pair of primers does not amplify alleles of closely related genes such as DQB2 (DXB) or the DPB1 and DRB genes.

8. A pair of oligonucleotide primers as claimed in claim 7 selected from the group of the primer pairs DB130 (SEQ ID NO:47)/DB131 (SEQ ID NO:48) and DB130 (SEQ ID NO:47)/GH29 (SEQ ID NO:70).

9. A kit for DQB1 DNA typing comprising a pair of oligonucleotide primers as claimed in claims 7 or 8.

10. The kit of claim 9 that further comprises a set of oligonucleotide probes, preferably including a probe selected from the group consisting of DB51, DB53, DB54, DB55, DB69, DB78, DB79, DB80, DB105, DB107, DB110, DB114, DB115, DB158, DB162, and UG82.

11. The kit of claim 10 that further comprises a thermostable DNA polymerase.

12. The kit of claim 11 that further comprises one or more deoxyribonucleoside 5'-triphosphates.

## Patentansprüche

1. Eine Methode zur Bestimmung der Nukleinsäuren des Typus DQB1 in einer Probe, wobei die Methode umfasst (a) die Amplifizierung von DQB1 Gensequenzen des zweiten Exons, ohne dass Allele der nah verwandten Gene wie DQB2 (DXB) oder der DPB1 und der DRB Gene mit amplifiziert werden, in Gegenwart eines Primers der für alle Allele des DQB1 Gens spezifisch ist; (b) Hybridisierung jeder amplifizierten Nukleinsäure mit einer Reihe von Oligonukleotidsonden unter Bedingungen bei denen nur Sonden, die an Sequenzen hybridisiert sind, die exakt komplementär sind, stabil an die amplifizierte DNA gebunden bleiben; und (c) Bestimmung ob Amplifikation erfolgt ist.

2. Die Methode gemäss Anspruch 1, worin der Primer für die Amplifikation ausgewählt ist aus der Gruppe bestehend aus DB130 (SEQ ID NO:47) und DB131 (SEQ ID NO:48), vorzugsweise das Primerpaar DB130 (SEQ ID NO:47)/DB131 (SEQ ID NO:48) oder das Primerpaar DB130 (SEQ ID NO:47) und GH29 (SEQ ID NO:70).

3. Die Methode nach Anspruch 1 oder 2, worin der Amplifikationsschritt mittels PCR durchgeführt wird.

4. Die Methode nach einem der Ansprüche 1 bis 3, worin die Reihe von Oligonukleotidsonden zwei oder mehr Sonden aus der Gruppe von Sonden bestehend aus DB51, DB53, DB54, DB55, DB69, DB78, DB79, DB80, DB105, DB107, DB110, DB114, DB115, DB158, DB162, und UG82 umfasst.

5. Die Methode gemäss Anspruch 4, worin die Sonden markiert sind.

6. Die Methode gemäss Anspruch 4 oder 5, worin die Reihe von Oligonukleotidsonden an einen festen Träger gebunden ist, und jede einzelne Sonde an einer bestimmten und unterscheidbaren Stelle auf dem festen Träger lokalisiert ist.

7. Ein Oligonukleotidprimerpaar das spezifisch ist für alle Allele des DQB1 Gens, wobei das Primerpaar Allele der nah verwandten Gene, wie die DQB2 (DXB) oder DPB1 und DRB Gene, nicht amplifiziert.

8. Ein Oligonukleotidprimerpaar gemäss Anspruch 7 ausgewählt aus der Gruppe der Primerpaare DB130 (SEQ ID NO:47)/DB131 (SEQ ID NO:48) und DB130 (SEQ ID NO:47)/GH29 (SEQ ID NO:70).

9. Ein Kit zur DQB1 DNA Typisierung das ein Primerpaar gemäss Anspruch 7 oder 8 enthält.

10. Das Kit gemäss Anspruch 9 das zusätzlich eine Reihe von Oligonukleotidsonden enthält, wobei vorzugsweise eine Sonde die ausgewählt ist aus der Gruppe bestehend aus DB51, DB53, DB54, DB55, DB69, DB78, DB79, DB80, DB105, DB107, DB110, DB114, DB115, DB158, DB162, und UG82 enthalten ist.

11. Das Kit gemäss Anspruch 10 das zusätzlich eine thermostabile DNA Polymerase enthält.

12. Das Kit gemäss Anspruch 11 das zusätzlich eines oder mehrere Desoxyribonukleosid 5'-triphosphate enthält.

## Revendications

1. Procédé pour la détermination du type DQB1 d'acides nucléiques dans un échantillon, lequel procédé comprend (a) l'amplification de séquences du second exon du gène DQB1, sans amplifier d'allèles de gènes étroitement apparentés tels que DQB2 (DXB) ou les gènes DPB1 et DRB, en présence d'une amorce spécifique de tous les allèles du gène DQB1; (b) l'hybridation de tout acide nucléique amplifié, à l'aide d'une série de sondes oligonucléotidiques, dans des conditions telles que seules les sondes hybridées avec des séquences exactement complémentaires peuvent rester liées de façon stable à l'ADN amplifié; et (c) l'étape consistant à déterminer si l'amplification s'est produite.

2. Procédé selon la revendication 1, dans lequel l'amorce utilisée pour l'amplification est choisie parmi DB130 (ID SEQ n° 47) et DB131 (ID SEQ n° 48), de préférence la paire d'amorces DB130 (ID SEQ n° 47)/DB131 (ID SEQ n° 48) ou la paire d'amorces DB130 (ID SEQ n° 47) et GH29 (ID SEQ n° 70).

3. Procédé selon la revendication 1 ou 2, dans lequel ladite étape d'amplification est effectuée par PCR (amplification en chaîne par polymérase).

4. Procédé selon l'une quelconque des revendications 1 à 3, dans lequel la série de sondes oligonucléotidiques comprend deux sondes ou plus choisies parmi DB51, DB53, DB54, DB55, DB69, DB78, DB79, DB80, DB105, DB107, DB110, DB114, DB115, DB158, DB162 et UG82.

5. Procédé selon la revendication 4, dans lequel lesdites sondes sont marquées.

6. Procédé selon la revendication 4 ou 5, dans lequel ladite série de sondes est immobilisée sur un support solide, et chaque sonde distincte est localisée en une localisation distincte et séparée sur ledit support solide.

7. Paire d'amorces oligonucléotidiques spécifiques de tous les allèles du gène DQB1, laquelle paire d'amorces n'amplifie pas d'allèles de gènes étroitement apprentés, tels que DQB2 (DXB) ou les gènes DPB1 et DRB.

8. Paire d'amorces oligonucléotidiques selon la revendication 7, choisie parmi les paires d'amorces DB130 (ID SEQ n° 47)/DB131 (ID SEQ n° 48) et DB130 (ID SEQ n° 47) et GH29 (ID SEQ n° 70).

9. Nécessaire pour le typage d'ADN de DQB1, comprenant une paire d'amorces oligonucléotidiques selon la revendication 7 ou 8.

10. Nécessaire selon la revendication 9, comprenant en outre une série de sondes oligonucléotidiques, de préférence comprenant une sonde choisie parmi DB51, DB53, DB54, DB55, DB69, DB78, DB79, DB80, DB105, DB107, DB110, DB114, DB115, DB158, DB162 et UG82.

11. Nécessaire selon la revendication 10, comprenant en outre une ADN polymérase thermostable.

12. Nécessaire selon la revendication 11, comprenant en outre un ou plusieurs désoxyribonucléoside 5'-triphosphates.
